# EUROPEAN PATENT APPLICATION

(11) **EP 2 201 904 A1**
(43) Date of publication of application: **30.06.2010**
(21) Application number: 08794083.9
(22) Date of filing: 28.07.2008
(51) Int. Cl.: A61B 18/12, A61N 1/32

(54) **METHOD FOR REMOVING TATTOOS AND SCARS**

(30) Priority: 02.10.2007 RU 2007136276
(71) Applicant: Avramenko, Konstantin Stanislavovich, Ul. Zavodskaya 9/1-16 Klimovsk Moskovskaya obl. 142181 (RU)
(72) Inventor: Avramenko, Konstantin Stanislavovich, Ul. Zavodskaya 9/1-16 Klimovsk Moskovskaya obl. 142181 (RU)
(74) Representative: Sergejeva, Valentina
(86) International application number: PCT/RU2008/000492
(87) International publication number: WO 2009/045124

(57) **Abstract**

The invention relates to medicine and can be used for removing tattoos, scars, keloid and wound cicatrices. The inventive method consists in acting on the epidermal layer with an alternating electric field in such a way that high-frequency discharges are formed between the epidermal layer and a needle electrode. Said method makes it possible to effectively removing tattoos of different colours and scars of different nature. The removal is carried out during a minimum time and does not bring about any complications, since the skin areas with defects to be removed are not exposed to excessive thermal action

## Description

The invention relates to medicine and can be used for removing tattoos, scars, and keloid and wound cicatrices.

A tattoo is a combination of small punctures or incisions between 0.1 mm and 4-5 mm deep on human skin that are filled with a colorant. As protection against foreign matter, such as the colorant introduced into the skin through the punctures or incisions, the human organism begins to build protective capsules of connective tissue around each particle of the colorant embedded in the skin. The capsules are fully formed within three to seven days after the colorant has been introduced into human skin through the punctures (incisions).

The protective capsules that prevent colorant particles from penetrating deeper into the organism make the tattoo very stable and difficult to remove.

There are several methods for removing tattoos:
1. Removing a tattooed skin patch mechanically by plastic surgery followed by pulling and stitching together the edges of the resultant wound, or, in the case of a large tattooed skin patch, transplanting a patch of clean skin from another part of the patient's body, or from a donor.
2. Etching or cauterizing the tattooed skin patch by acids or alkalis.
3. Removing the tattooed skin patch mechanically by a cutter using the Dermatom device.
4. Cauterizing the tattooed skin patch by any heated instrument.
5. Cauterizing or vaporizing the tattooed skin patch by a CO₂ laser.
6. Exposing particles of the tattoo colorant within the patient's skin successively, during several sessions, to the radiation of erbium, neodymium, ruby, Q-Switch, and similar lasers. In this method, laser radiation does not damage the patient's skin, and penetrates through the skin and heats the darker particles of the tattoo colorant within the skin.

There also are the following methods for removing scars:
1. Removing a scar surgically, the entire scar at one time or in a small strip 2 to 3 cm wide and no more than 6 to 9 cm long, followed by stitching the edges of the resultant cut. The healthy skin stretches to cover the patch of the scar cut out. This method also includes skin transplantation from the patient's or donor's body to the patch of the scar cut out.
2. Laser scar polishing. Similar to multiple sessions to remove tattoos by erbium lasers. Each time, laser vaporizes the top layer of the scar between 0.1 and 0.3 mm thick only. This makes the scar itself flatter by exactly this thickness.
3. Using injections of various biologically active substances that "loosen" the scar slightly and flatten it.

Similar methods for removing tattoos or scars are disclosed in patent publications as well (*see:* RU 2,092,119, RU 2,247,554, and RU 2,044,552),

The above methods are disadvantageous because they are incapable of removing a scar completely, but only reduce slightly the convexity and hardness of the scar, or damage the skin thermally as tattoos are removed, leaving scars as a result.

When craters are cauterized to vaporize skin particles containing tattoo colorant by CO₂ lasers, laser radiation heat propagates into the underlying tissues to a depth of 3 to 5 times the depth of a crater cauterized into tissues under and around the crater. This method causes complete thermal damage and destruction of tissues under and around the crater to 3-5 times the depth of the crater cauterized.

Subsequently, the destroyed tissues are replaced on healing with connective cicatricose tissue that forms unaesthetic cicatrices and scars in the arca where the tattoo has been removed.

Comparison with tattoo removal by the laser method shows that erbium, neodymium, ruby, Q-Switch, and other lasers require a large number of sessions to treat a patient's tattooed skin - at least 5 sessions, and ordinarily 10 to 30 sessions. The time interval between the sessions is at least 3 weeks. Accordingly, the tattoo removal time in, for example, 10 sessions is at least 6 to 8 months long, sometimes from 2 to 3 years.

When this method is used, a tattoo of a small area is removed in a single session.
After the craters have healed within 2 to 3 weeks, no tattoo is in evidence any more.

Yet another negative side of the laser method is that red tattoo ink cannot be removed completely because laser wavelengths are chosen to avoid destruction of blood erythrocytes in skin capillaries upon penetration deep into the skin to reach tattoo colorant particles. Erythrocytes mostly have a red color because of hemoglobin. Accordingly, when the colorant is red or orange, the laser produces no effect, and only has an insignificant effect on a yellow colorant.

When, therefore, lasers of the above types are used to remove tattoo colourants of orange and yellow color, a much larger number of sessions, up to 40 or 50, is required. Tattoos of red color are impossible to remove at all.

The technical result of this invention is that skin defects are removed without damaging tissues under and around the defects and without producing cicatrices or scars.

This technical result is achieved by a method for removing skin defects such as tattoos and scars, wherein the defect areas of the epidermal layer are cauterized using a source of highly concentrated energy. In accordance with the invention, an alternating electric field is used to produce high-frequency discharges between the epidermal layer and a needle electrode.

Furthermore, it is reasonable to keep the spacing between the electrode and the epidermal layer within 0.01 mm to 20 mm, the preferable spacing between the electrode and the epidermal layer being from 0.5 mm to 4.5 mm.

The number of discharges per second as a tattoo is removed can be varied between 1 and 1,000,000.

It is preferred to cauterize epidermal layer areas of a diameter that docs not exceed 10 mm, distributed uniformly over the defect area. Cauterization of the remaining defect areas is to be repeated upon healing, again and again until the defect is cauterized completely over the entire area thereof.

In addition, it is preferred, prior to cauterization of the epidermal layer, to cool it by between 5°C and 50°C.

The high-frequency discharge has a frequency of 20 to 10,000 V.

The idea of the invention is as follows:

The claimed method for removing tattoos and scars consists in using a source of high-frequency voltage having a single needle electrode to remove defective skin patches. For example, a source suited for this purpose is the device disclosed in Patent RU No. 2,191,113 in which the needle electrode extends beyond the edges of the nozzle opening. The device operates without any gas supplied thereto. A quasi-static electric field is produced on the needle electrode. The operator causes the needle electrode of the emitting head to move toward a tattooed skin patch to be removed to a distance of 0.01 mm to 20 mm. When the quasi-static charges on the needle electrode reach a density sufficient for sparking over the spacing between the patient's skin and the needle electrode point, a quasi-static discharge occurs.

Discharges of this type occur at a rate of 1 to 1,000,000 per second, depending on the electric power supplied to the emitting head and the spacing between the needle electrode point and the patient's skin.

When this situation develops, the needle electrode point and the patient's skin surface form a system similar to a vacuum diode.

As a positive half-wave of the alternating quasi-static electric field flows, it sparks over from the needle electrode point that is identical to the anode in a vacuum diode to the patient's skin.

When a negative half-wave of the alternating quasi-static electric field flows to the needle electrode point, no spark-over occurs from the patient's skin to the needle electrode. The alternating quasi-static electric field is rectified in this case.

Each single electric discharge sparking over from the needle electrode to the patient's skin burns a microscopic crater in the patient's skin by vaporizing and burning all organic matter, both the skin cells and particles of any tattoo colorant, at the point of impact.

Since, however, such discharges follow at a rate of up to 1,000,000 per second, the total effect of this multitude of discharges leaves a clearly visible crater 0.1 mm to 5-6 mm deep in the patient's skin. As this multiple crater, though, consists of a great number of micro-craters, multiple thermal damage does not spread deep into the skin or sideways from the edges of the total crater.

When a high-frequency discharge is used to remove tattoos using a needle electrode, its energy does not propagate deep into the tissues from the crater it has formed. Thermal damage does not affect the surrounding tissues to a distance in excess of 0.3 mm (typically, between 0.05 mm and 0.1 mm).

Accordingly, the damaged tissues are not replaced with connective tissue as they are in the case of the CO₂ laser, and no cicatrices and scars develop.

The claimed device is specific in yet another physical respect in use.

Electric charges sparking over from the needle electrode to the patient's skin are quasi-static, that is, completely similar to a static electric discharge.

It is common knowledge that static electricity can only propagate over the surface of a conductor or semiconductor, without penetrating deep inside. Accordingly, when the patient's skin is treated with such quasi-static discharges, they cannot, because of their nature, penetrate deep into the patient's skin and inflict damage to any underlying and sideways tissues.

For this reason, the cosmetic effect of the claimed method when it is used to remove tattoos and scars is incomparably more favorable than that of all the above-listed methods for removing tattoos and scars.

A high-frequency discharge used to remove tattoos leaves no scars at all. As the craters heal, the patient's organism regenerates smooth skin at the site of a crater, with the structure of capillary lines restored. The hair, too, is fully restored over time.

The color and chemical composition of the tattoo colorant are of no significance at all for tattoos to be removed in accordance with the claimed method.

Particularly good results were obtained by the claimed method in removing tattoos and scars in small patches of 2 sq.cm. in area at most, rather that those covering a large continuous area. Typically, the diameter of a patch to be removed is between 4 and 5 mm, but no longer than 10 mm. Patches of this type are distributed uniformly, for example, in a chessboard pattern, over the entire area of a defect. After the patches removed have healed (within 1 to 2 weeks), the patches on the remaining portion of the tattoo or scar are removed in a similar manner, and this pattern is followed until the defect has been removed completely over the entire area.

In this case, several positive results are attained at the same time:
1. In the event of accidental mechanical damage by an external factor, it only affects 1 to 3 small craters, rather than the entire area if the tattoo is removed in large patches.
2. In the event of infection during post-operation care, festering affects one or several small patches removed, rather than the full area of a patch if the tattoo is removed over larger areas.
3. Untreated skin patches between the patches removed impart elasticity to the entire structure, whereas tattoo removal over large areas results in the formation of one continuous scab that can burst under the tension of the patient's subcutaneous muscles; the patient has a discomforting sense of skin shrinkage and limited mobility in the spot from where the tattoo has been removed.
4. Small patches removed heal significantly faster than a single patch of large area of the tattooed skin removed.

A particularly positive cosmetic effect is produced by lowering the temperature of the skin patch to be treated by at least 10 to 20 degrees Centigrade, or more, before the start of the operation to remove tattoos or scars by the claimed method.

In all, over 300 patients were treated to optimize the operating mode of the tattoo removing device.

First, studies were conducted to find the relationship between the quality of tattoo removal and the frequency of the converter of the device used for its purpose.

The frequency of the high-voltage resonance transformer used in the device was varied for the purposes of the studies.

Data obtained for 73 instances of device application showed that variations in the frequency of the electronic converter supplying the resonance transformer within the range of 1,000 Hz to 1,000,000 Hz do not affect significantly the quality of tattoo removal.

When, however, higher frequencies are used, the size and cost of the resonance transformer decrease. The frequency above 50,000 Hz, therefore, is an optimal choice for reducing the cost of the device.

The effect of the high-frequency discharge generated by the device used was also studied.

In all, 127 experiments were carried out.

The results received show that a voltage in excess of 10,000 V between the needle electrode and the patient's skin may cause burns developing into cicatrices on the skin.

Studies were also performed to optimize the spacing between the needle electrode and the patient's skin (in 239 experiments).

The results of the studies show that a spacing no more than 4 to 5 mm between the needle electrode and the patient's skin is an optimal choice. The spacing increasing over 5 mm to 20 mm caused electrostatic discharge "scattering," making it difficult to point the discharge at the desired tattoo patch, and the total area exposed to the discharge expanded to clean skin areas surrounding the tattoo as well.

When the spacing between the needle electrode and the patient's skin was increased to over 20 mm, the electric power required for the discharge to spark over this spacing was so high that it caused burns developing into cicatrices and scars in 100% of the cases (17 experiments were conducted).

It is obvious, therefore, that the optimal spacing between the needle electrode and the patient's skin is between 0.01 mm and 5 mm. The maximum permissible spacing is not to exceed 20 mm.

Cicatrices and scars of different etiology were removed from patients' skin in 47 experiments conducted for this purpose.

Very favorable results from the cosmetic viewpoint - scars vanished altogether - were received in 34 cases.

Insignificant smoothing of scars treated was achieved in seven cases.

No changes occurred in six cases.

It follows from the materials described that the claimed method for removing tattoos and scars helps effectively to remove tattoos of different colors and scars of different nature within the shortest possible time period, without causing complications because the skin patches with defects to be removed are not exposed to excessive thermal effects.

## Claims

1. A method for removing skin defects in the form of tattoos and scars by cauterizing the defective patches of the epidermal layer by exposing them to a source of highly concentrated energy, wherein the defects are exposed to an alternating electric field producing high-frequency discharges between the epidermal layer and a needle electrode.

2. A method as claimed in claim 1, wherein the spacing between the electrode and the epidermal layer varies between 0.01 mm and 20 mm.

3. A method as claimed in claim 1, wherein the discharge voltage ranges from 20 V to 10,000 V.

4. A method as claimed in claim 1, wherein exposure is effected by discharges sparking over at a rate of 1 to 1,000,000 discharges per second.

5. A method as claimed in claim 1, wherein the patches of the epidermal layer to be cauterized have a diameter within 10 mm and are distributed uniformly over the area of the defect, the remaining defect patches being cauterized after the preceding patches have healed, said steps being repeated until the entire area of the defect has been cauterized completely.

6. A method as claimed in claim 1, wherein the epidermal layer is cooled by 5°C to 50°C before exposure to discharges.
